(19) Europäisches Patentamt
European Patent Office
Office européen des brevets

(11) **EP 1 784 375 B1**

(12) **EUROPÄISCHE PATENTSCHRIFT**

(45) Veröffentlichungstag und Bekanntmachung des Hinweises auf die Patenterteilung:
**19.05.2010 Patentblatt 2010/20**

(21) Anmeldenummer: **05782941.8**

(22) Anmeldetag: **22.08.2005**

(51) Int Cl.:
*C07C 51/02* (2006.01)    *C07C 51/09* (2006.01)
*C07C 53/02* (2006.01)

(86) Internationale Anmeldenummer:
**PCT/EP2005/009066**

(87) Internationale Veröffentlichungsnummer:
**WO 2006/021411 (02.03.2006 Gazette 2006/09)**

(54) **VERFAHREN ZUR HERSTELLUNG VON AMEISENSÄURE**

METHOD FOR THE PRODUCTION OF FORMIC ACID

PROCEDE DE PRODUCTION D'ACIDE FORMIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.08.2004 DE 102004040789**

(43) Veröffentlichungstag der Anmeldung:
**16.05.2007 Patentblatt 2007/20**

(73) Patentinhaber: **BASF SE**
**67056 Ludwigshafen (DE)**

(72) Erfinder:
• **KARL, Jörn**
**67063 Ludwigshafen (DE)**
• **HAUK, Alexander**
**67071 Ludwigshafen (DE)**

(56) Entgegenhaltungen:
**EP-A- 0 563 831    US-A- 1 919 850**
**US-A- 2 511 198    US-A- 4 218 568**

• **HUNTSMAN: "Technical Bulletin, Amine Applications and Properties data," * Seiten 1,5 ***
• **"Aldrich Katalog 1996-1997" * Seite 1072 ***
• **"Dissociation Constants of Organic Acids and Bases, Sektion 8, CRC Handbook of Tables for Organic Compound Identification, 76th Edition, 1995-1996," CRC PRESS**
• **"Physical Constants of Organic Compounds, Sektion 3, Nr., CRC Handbook of Tables for Organic Compound Identification, 76th Edition, 1995-1996" CRC PRESS**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von Ameisensäure durch thermische Zersetzung von Ammoniumformiaten tertiärer Amine.

Die Herstellung von Ameisensäure durch thermische Zersetzung von Ammoniumformiaten tertiärer Amine A gemäß der folgenden Reaktionsgleichung

$$HCOO^-HA^+ \rightleftharpoons HCOOH + HA$$

ist grundsätzlich bekannt. So beschreibt beispielsweise die EP-A 1432 ein Verfahren, bei dem man zunächst ein Ammoniumformiat eines tertiären Amins herstellt, indem man Methylformiat in Gegenwart eines tertiären Amins, welches bei Normaldruck bei wenigstens 180 °C siedet, in einer Menge von 0,5 bis 3,0 mol pro Mol Methylformiat, hydrolysiert. Als hierfür geeignete tertiäre Amine werden imldazolverbindungen vorgeschlagen. Eigene Untersuchungen der Anmelderin haben jedoch gezeigt, dass diese Imidazolverbindungen unter Prozessbedingungen nicht ausreichend stabil sind. Zudem verfärbt sich die so hergestellte Ameisensäure beim Stehen.

Die EP-A 563831 schlägt zur Lösung der im Verfahren der EP-A 1432 auftretenden Probleme vor, die thermische Spaltung in Gegenwart von sekundären Formamiden durchzuführen, wobei die Formamide 30 bis 150 K niedriger sieden sollen als das im Ammoniumformiat vorliegende tertiäre Amin. Bei den tertiären Aminen handelt es sich um hochsiedende Substanzen mit einem Siedepunkt bei Normaldruck von oberhalb 200 °C. Die nach diesem Verfahren erhaltene Ameisensäure neigt zudem ebenfalls zu Verfärbungen.

Der vorliegenden Erfindung liegt daher die Aufgabe zugrunde, ein Verfahren zur Herstellung von Ameisensäure bereitzustellen, bei dem eine Ameisensäure erhalten wird, die nicht oder in sehr viel geringerem Ausmaße als im Stand der Technik zu Verfärbungen neigt. Das Verfahren soll zudem wirtschaftlich sein, d. h. es solite insbesondere mit hohen Ausbeuten bezüglich der Einsatzstoffe, guten Raum-Zeitausbeuten und geringem Energiebedarf durchgeführt werden können.

Es wurde überraschenderweise gefunden, dass diese Aufgaben gelöst werden können durch thermische Spaltung von Ammoniumformiaten tertiärer Amine A, die bei Normaldruck einen Siedepunkt im Bereich von 105 bis 175 °C und einen $pK_a$-Wert in Wasser bei 25 °C im Bereich von 4 bis 9 aufweisen.

Demnach betrifft die vorliegende Erfindung ein Verfahren zur Herstellung von Ameisensäure durch thermische Spaltung von Ammoniumformiaten tertiärer Amine A, das dadurch gekennzeichnet ist, dass das dem Ammoniumformiat zugrundeliegende tertiäre Amin A einen Siedepunkt bei Normaldruck im Bereich von 105 bis 175 °C und einen $pK_a$-Wert in Wasser bei 25 °C im Bereich von 4 bis 9 aufweist, und dass man das Ammoniumformiat durch Hydrolyse von Methylformiat in Gegenwart des tertiären Amins A bereitstellt.

Der Begriff "tertiäres Amin" umfasst hier und im Folgenden sowohl tertiäre Amine, die wenigstens ein tertiäres Stickstoffatom aufweisen, das drei aliphatische oder cycloallphatlsche Substituenten aufweist, die gegebenenfalls mit dem Stickstoffatom ein mono- oder bicyclisches Ringgerüst bilden, als auch Stickstoffverbindungen, in denen das tertiäre Stickstoffatom in ein aromatisches Ringgerüst eingebunden ist.

Geeignete Amine sind solche, deren $pK_a$-Wert in Wasser bei Normaldruck und Normaltemperatur im Bereich von 4 bis 9 liegt. Bezüglich der Definition der $pK_a$-Werte sei an dieser Stelle auf Landoldt-Bömstein, 6. Auflage, Band II, S. 900 ff. verwiesen. Die Amine A weisen üblicherweise 1 oder 2 tertiäre Stickstoffatome, insbesondere 1 Stickstoffatom, und 6 bis 10 C-Atome sowie gegebenenfalls 1 oder 2 Sauerstoffatome auf.

Unter den tertiären Aminen sind Pyridinverbindungen, insbesondere Mono-, Di- und Tri-$C_1$-$C_4$-alkylpyridine mit vorzugsweise insgesamt 6 bis 10 C-Atomen, wie α-Picolin, β-Picolin, γ-Picolin, 2,4,6-Trimethylpyridin, 2-tert.-Butylpyridin, 2,6-Dimethylpyridin, 2,4-Dimethylpyridin, sowie Mono-$C_1$-$C_4$-alkoxypyridine bevorzugt.

Geeignet sind auch gesättigte, 5-, 6-, 7- oder 8-gliedrige Stickstoffheterocyclen, die an dem wenigstens einen Stickstoffatom eine N-$C_1$-$C_4$-Alkylgruppe tragen und die auch ein Sauerstoffatom als Ringglied aufweisen können wie N-Methylmorpholin, N-Ethylmorpholin und dergleichen.

Das zur thermischen Spaltung eingesetzte quartäre Ammoniumformiat kann nach an sich bekannten Verfahren hergestellt werden:

   i) durch direkte Umsetzung des entsprechenden tertiären Amins A mit Ameisensäure in Analogie zu den in GB-A 1028930 oder US 3,414,610 beschriebenen Verfahren,

   ii) durch übergangsmetallkatalysierte Hydrierung von Kohlendioxid zu Ameisensäure in Gegenwart des tertiären Amins A in Analogie zu den in EP-A 95321, EP-A 151510 oder EP-A 357243 beschriebenen Verfahren,

   iii) durch Umsetzung von Methylformiat mit Wasser und anschließende Extraktion der gebildeten Ameisensäure mit dem tertiären Amin A in Analogie zu der in DE-A 3428319 Methode oder

   iv) durch Umsetzung von Methylformiat mit Wasser in Gegenwart des tertiären Amins in Analogie zu der Vorschrift der EP-A 1432.

Die nach den Verfahren i) bis iv) erhaltenen Rohprodukte enthalten neben dem jeweiligen Ammoniumformiat in Abhängigkeit von dem jeweils gewählten Verfahren weitere Verbindungen, beispielsweise organische Lösungsmittel, nicht umgesetzte Einsatzmaterialien, beispielsweise Methylformiat, gebildete Koppelprodukte, beispielsweise Methanol, und/oder Katalysatorkomponenten. So enthalten beispielsweise die nach dem Verfahren iv) erhaltenen Reaktionsgemische neben dem Ammoniumformiat des zur Hydrolyse eingesetzten tertiären Amins A als weitere Verbindungen in der Regel noch Wasser, Methanol und/oder Methylformiat.

Es empfiehlt sich, diese Bestandteile vor der erfindungsgemäßen thermischen Zersetzung des Ammoniumformiats weitgehend vorzugsweise vollständig oder nahezu vollständig, insbesondere zu wenigstens 99 % und besonders bevorzugt zu wenigstens 99,9 % und speziell zu wenigstens 99,95 %, bezogen auf die Gesamtmenge der erhaltenen Verunreinigungen zu entfernen bzw. auf einen Restgehalt von weniger als 1 Gew.-%, insbesondere weniger als 0,1 Gew.-% und speziell auf 100 bis 500 ppm, bezogen auf das der thermischen Spaltung zugeführte Ammoniumformiat zu verringern. Angaben in ppm sind hier und im Folgenden als $10^{-6}$ Gewichtsanteile zu verstehen.

Zur thermischen Spaltung der Ammoniumformiate wird man in der Regel eine Ammoniumformiat-haltiges Rohprodukt, gegebenenfalls nach weitgehender Abtrennung von nicht umgesetzten Edukten und Koppelprodukten oder sonstigen flüchtigen Verunreinigungen, wie beispielsweise nicht umgesetztes Methylformiat, Methanol und gegebenenfalls Wasser, auf eine Temperatur erhitzen, bei der eine Spaltung des Ammoniumformiats in Ameisensäure und tertiäres Amin A stattfindet. Vorzugsweise wird man die thermische Spaltung so führen, dass die Temperatur der Mischung 130 °C und insbesondere 125 °C nicht überschreitet und insbesondere im Bereich von 90 bis 125 °C liegt. Die so freigesetzte Ameisensäure wird in üblicher Weise aus dem Reaktionsgemisch entfernt. Zweckmäßigerweise erfolgt das Entfernen der Ameisensäure durch Abdestillieren der Ameisensäure, gegebenenfalls zusammen mit Wasser, aus der eingesetzten Reaktionsmischung. Um ein Abdestillieren der Ameisensäure aus dem Reaktionsgemisch zu gewährleisten, erfolgt die thermische Spaltung des Ammoniumformiats vorzugsweise bei einem Druck von nicht mehr als 1,1 bar, beispielsweise im Bereich von 0,01 bis 1 bar, insbesondere im Bereich von 0,02 bis 0,8 bar und besonders bevorzugt im Bereich von 0,05 bis 0,5 bar.

Zur thermischen Spaltung des Ammoniumformiats eignen sich grundsätzlich vor allem Destillationsapparaturen wie Destillationskolonnen, beispielsweise Füllkörper- und Glockenbodenkolonnen. Als Füllkörper eignen sich insbesondere Packungen, Glas-Raschig- und Pall-Ringe, wobei zur Vermeidung von Korrosion keramische Füllkörper bevorzugt sind.

In einer Ausführungsform des erfindungsgemäßen Verfahrens führt man die thermische Spaltung in Gegenwart eines Formamids eines sekundären Amins durch. Geeignete Formamide sind insbesondere die in EP-A-563831 genannten Verbindungen der allgemeinen Formel II, insbesondere solche, deren Siedepunkt bei Normaldruck 170 °C nicht überschreitet. Beispiele für derartige Formamide sind insbesondere N-Formylmorpholin, N-Formylpyrrolidin, N-Formylpiperidin, N,N-Dimethylformamid und N,N-Diethylformamid. In einer anderen bevorzugten Ausführungsform führt man die thermische Spaltung in Abwesenheit des Formamids durch.

Vorteilhafterweise gestaltet man das erfindungsgemäße Verfahren als kontinuierlichen Prozess aus. Hierzu wird der Reaktoraustrag der Ammoniumformiat-Synthese, der in der Regel noch flüchtige Bestandteile enthält, zweckmäßigerweise einer ersten Destillationsapparatur zugeführt, wobei die Hauptmenge an flüchtigen Bestandteilen, vorzugsweise bis zu einem Restgehalt von nicht mehr als 1 Gew.-%, insbesondere nicht mehr als 0,1 Gew.-% und speziell nicht mehr als 500 ppm über Kopf abdestilliert wird. Der Sumpfabzug der ersten Destillationsapparatur wird dann einer zweiten Destillationsapparatur zur thermischen Spaltung des Ammoniumformiats zugeführt. In dieser zweiten Destillationsapparatur herrschen die oben für die Ammoniumformiat-Spaltung angegebenen Druckverhältnisse. Die Temperatur des Kolonnensumpfs weist die oben zur thermischen Spaltung des Ammoniumformiats angegebenen Temperaturen auf und liegt insbesondere im Bereich von 90 bis 120 °C. In dieser zweiten Destillationsapparatur, die zweckmäßigerweise als Kolonne mit vorzugsweise 15 bis 30 und Insbesondere mit 20 bis 25 theoretischen Böden ausgestaltet ist, wird dann Ameisensäure und gegebenenfalls Wasser über Kopf abdestilliert. Der Kolonnensumpf enthält im Wesentlichen das eingesetzte tertiäre Amin A mit gegebenenfalls Resten an Ameisensäure. Der Sumpf wird daher zweckmäßigerweise in den Herstellungsprozess des Ammoniumformiats zurückgeführt.

In einer bevorzugten Ausgestaltung des erfindungsgemäßen Verfahrens setzt man zur thermischen Spaltung ein Ammoniumformiat ein, das man durch Hydrolyse von Methylformiat in Gegenwart des tertiären Amins A hergestellt hat. Demnach umfasst eine bevorzugte Ausführungsform des erfindungsgemäßen Verfahrens die Hydrolyse von Methylformiat in Gegenwart des tertiären Amins A und die thermische Spaltung des dabei erhaltenen Ammoniumformiats des tertiären Amins A.

Die Hydrolyse erfolgt in der Regel durch Umsetzung von Methylformiat mit Wasser, in Gegenwart von 0,5 bis 3 mol, vorzugsweise 0,8 bis 1,5 moi und insbesondere 0,9 bis 1,2 mol tertiärem Amin A, bezogen auf 1 mol Methylformiat. Sofern das Amin A im Unterschuss, bezogen auf das Methylformiat eingesetzt wird, liegt im Reaktionsgemisch eine Mischung aus Ameisensäure und Ammoniumformiat vor, das zur thermischen Spaltung eingesetzt werden kann, insbesondere wenn man keine wasserfreie Ameisensäure herstellen will.

Die zur Erzielung ausreichender Umsätze erforderliche Menge an Wasser beträgt in der Regel wenigstens 0,8 mol pro Mol Methylformiat und liegt üblicherweise im Bereich von 0,8 bis 1,2 mol Wasser pro Mol Methylformiat.

In der Regel erfolgt die Hydrolyse bei Temperaturen im Bereich von 50 bis 200 °C, vorzugsweise im Bereich von 70 bis 160 °C. Aufgrund der Flüchtigkeit des Methylformiats - der Siedepunkt liegt bei Normaldruck bei 32 °C - erfolgt die Hydrolyse in der Regel unter erhöhtem Druck, üblicherweise bei einem Druck im Bereich von 1,1 bis 50 bar, insbesondere im Bereich von 2 bis 30 bar und speziell im Bereich von 3 bis 20 bar. Üblicherweise führt man die Hydrolyse bis zu einem Umsatz von wenigstens 50 %, bezogen auf eingesetztes Methylformiat und insbesondere bis zu einem Umsatz von 60 bis 95 %. Dementsprechend enthält das Reaktionsprodukt neben dem bei der Reaktion gebildeten Ammoniumformiat und Methanol noch nicht umgesetztes Methylformiat und gegebenenfalls Wasser.

Es empfiehlt sich, nicht umgesetztes Methylformiat und Methanol vor der thermischen Spaltung des Ammoniumformiats aus der Reaktionsmischung zu entfernen. Dies erfolgt üblicherweise durch destillative Aufarbeitung des Reaktionsgemischs, wobei man zumindest die Hauptmenge des bei der Reaktion gebildeten Methanols und des gegebenenfalls in der Reaktionsmischung vorhandenen Methylformiats, vorzugsweise bis zu einem Restgehalt dieser Bestandteile von nicht mehr als 0,1 Gew.-% und insbesondere nicht mehr als 0,5 ppm aus der Reaktionsmischung abdestilliert und den erhaltenen Destillationsrückstand, welcher im Wesentlichen aus Ammoniumformiat, gegebenenfalls Ameisensäure und Wasser besteht, anschließend der thermischen Spaltung zuführt.

Zur Durchführung der Hydrolyse kann man beispielsweise so vorgehen, dass man zunächst Methylformiat und Wasser und einen Teil, beispielsweise 20 bis 80 %, des Amins unter Reaktionsbedingungen miteinander umsetzt und anschließend die Restmenge an Amin zusetzt, bis der gewünschte Umsatz erreicht ist. Vorzugsweise setzt man jedoch Methylformiat, Wasser und Amin in dem gewünschten Mengenverhältnis miteinander um.

Vorteilhafterweise gestaltet man Hydrolyse und anschließende thermische Spaltung als kontinuierlichen Prozess aus. Hierzu wird zunächst das Methylformiat zusammen mit der gewünschten Menge an Wasser und tertiärem Amin A einer ersten Reaktionszone unter den oben bezeichneten Reaktionsbedingungen zugeführt. Die Verweilzeit in der ersten Reaktionszone wird so gewählt, dass die oben genannten Umsätze erreicht werden. Der Reaktoraustrag der Hydrolysereaktion, der neben Methanol in der Regel noch Methylformiat und Wasser als weitere Bestandteile enthält, wird zweckmäßigerweise einer ersten Destillationsapparatur zugeführt, wobei die Hauptmenge an Methylformiat und Methanol, vorzugsweise bis zu einem Restgehalt von nicht mehr als 0,1 Gew.-% und insbesondere nicht mehr als 500 ppm, z. B. bis zu einem Restgehalt von 100 bis 500 ppm über Kopf abdestilliert werden. Die erste Destillationsapparatur ist in der Regel als Kolonne mit 20 bis 30 theoretischen Böden ausgestaltet. Der Fachmann wird dabei den Druck so wählen, dass die Sumpftemperatur in der ersten Destillationsapparatur vorzugsweise 130 °C und insbesondere 125 °C nicht überschreitet. Üblicherweise beträgt der Druck nicht mehr als 1,1 bar. Der Sumpfabzug der ersten Destillationskolonne wird dann, wie oben beschrieben, einer zweien Destillationsapparatur zur thermischen Spaltung des Ammoniumformiats zugeführt, wobei man Ameisensäure und gegebenenfalls Wasser über Kopf abdestilliert. Der Sumpf kann dann der Hydrolysereaktion wieder zugeführt werden. Bezüglich der Druck- und Temperaturverhältnisse in der zweiten Kolonne gelten obige Angaben zur thermischen Spaltung entsprechend.

Das Destillat der ersten Kolonne kann, sofern es noch Methylformiat enthält, einer weiteren Destillation zur Trennung von Methanol und Methylformiat unterworfen werden. Das dabei anfallende Methylformiat kann ebenfalls in die Hydrolysereaktion zurückgeführt werden.

Das erfindungsgemäße Verfahren erlaubt eine effiziente Herstellung von Ameisensäure, die auch beim längeren Stehen im Unterschied zu der nach dem Stand der Technik erhaltenen Ameisensäure nicht oder nur in sehr viel geringerem Umfang zur Verfärbung neigt. Zudem ist das Verfahren im Vergleich zu den bekannten Verfahren wirtschaftlicher, da die zur thermischen Spaltung erforderlichen Temperaturen niedriger sind als im Stand der Technik. Das Verfahren liefert Ameisensäure in hoher Ausbeute, bezogen auf die Einsatzmaterialien und kann effizient mit hohen Raum/Zeit-Ausbeuten durchgeführt werden. Da eine Rückführung der eingesetzten Base möglich ist, können die Kosten für das Verfahren weiter gesenkt werden.

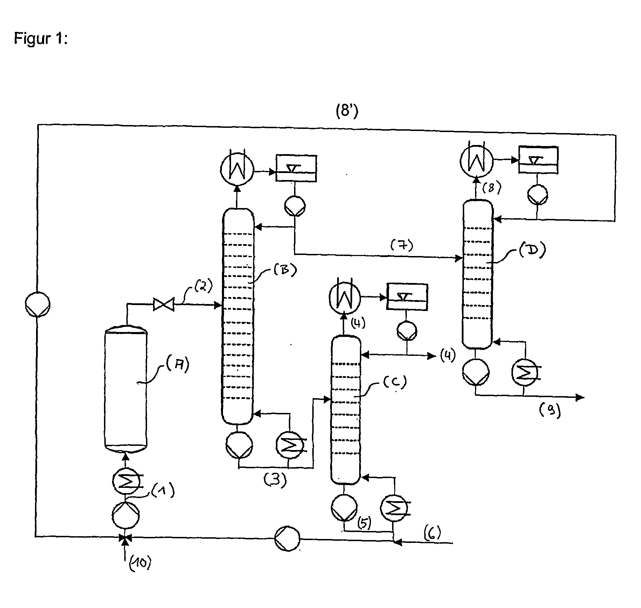

In der folgenden Figur 1 und in dem Beispiel ist das erfindungsgemäße Verfahren näher erläutert.

Figur 1 zeigt schematisch eine kontinuierliche Ausgestaltung des erfindungsgemäßen Verfahrens, bei dem man das Ammoniumformiat durch Hydrolyse von Methylformiat mit Wasser in Gegenwart des tertiären Amins herstellt. Hierzu wird als Stoffstrom 1 Wasser, Methylformiat und tertiäres Amin in den Hydrolysereaktor A eingespeist. Der Reaktoraustrag wird entspannt und in die Destillationskolonne B eingespeist. Der Destillationssumpf der Kolonne B, welcher Ammoniumformiat und Wasser enthält, wird als Stoffstrom 3 in eine weitere Destillationskolonne C eingespeist. In Kolonne C findet die thermische Spaltung des Ammoniumformiats statt, wobei am Kopf der Kolonne wasserhaltige Ameisensäure in einer Konzentration von 70 bis 85 Gew.-% (Stoffstrom 4) abgezogen wird. Der Destillationssumpf der Kolonne C, der im wesentlichen das tertiäre Amin A und geringe Mengen an Ameisensäure enthält, wird als Stoffstrom 5 abgezogen, gegebenenfalls mit frischer Base (Stoffstrom 6) angereichert und mit den Einsatzmaterialien Wasser und Methylformiat (Stoffstrom 10) vereinigt und als Stoffstrom 1 in die Hydrolysereaktion zurückgeführt.

Am Kolonnenkopf B wird das im Reaktoraustrag (2) der Hydrolyse A enthaltene Methanol sowie gegebenenfalls nicht umgesetztes Methylformiat abgezogen und als Stoffstrom (7) einer destillativen Trennung in Methylformiat und Methanol (Kolonne D) zugeführt. Das nicht umgesetzte Methylformiat wird am Kopf der Kolonne D als Stoffstrom (8) abgezogen und via (8') mit dem Stoffstrom der Einsatzmaterialien (Stoffstrom 1) vereinigt und so in die Hydrolyse A zurückgeführt. Als Sumpf der Kolonne D fällt im wesentlichen reines Methanol an, das als Stoffstrom 9 ausgeschleust und anderen Verwendungen, z.B. der Herstellung von Methylformiat, zugeführt werden kann.

Beispiel: Allgemeine Vorschrift für die Hydrolyse von Methylformiat und anschließende thermische Zersetzung des erhaltenen Ammoniumformiats

In einem kontinuierlich betriebenen Rührgefäß mit einem Reaktorinhalt von 200 ml wurden mit einer Zufuhrrate von 570 g/h eine Mischung aus Methylformiat, Wasser und 2,6-Dimethylpyridin im Molverhältnis 1 : 1 : 0,9 eingespeist. Die Reaktortemperatur lag bei 120 °C und der Druck bei 12 bar.

Das Reaktionsgemisch wurde in die Mitte einer Glockenbodenkolonne mit einem Durchmesser von 30 mm und einer Höhe von 3 m (30 Glocken) entspannt, so dass bei einer Kopftemperatur von 43 °C und einem Rücklaufverhältnis von 2 ein Gemisch aus 78 g/h Methanol und 56 g/h Methylformiat abgezogen wurde.

Das Sumpfprodukt der ersten Kolonne wurde in eine bei 400 mbar betriebene Füllkörperkolonne (Durchmesser 30 mm, Höhe 2 m, Rücklaufverhältnis 3) gegeben. Die Sumpftemperatur betrug etwa 125 °C. Bei einer Kopftemperatur von 79,5 °C destillierten 120 g/h einer Mischung aus Ameisensäure und Wasser mit einem Ameisensäuregehalt von 85 % ab. Als Sumpfprodukt fiel das tertiäre Amin an, das bis zu etwa 3 Gew.-% Ameisensäure enthielt. Dieses wurde quantitativ in die Hydrolysereaktion zurückgeführt.

Die erhaltene Ameisensäure hatte eine geringe Farbzahl die auch nach mehreren Tagen Lagerung nicht anstieg.

## Patentansprüche

1. Verfahren zur Herstellung von Ameisensäure durch thermische Zersetzung von Ammoniumformiaten tertiärer Amine A, **dadurch gekennzeichnet, dass** das tertiäre Amin A einen Siedepunkt bei Normaldruck im Bereich von 105 bis 175 °C und einen $pK_a$-Wert in Wasser bei 25 °C im Bereich von 4 bis 9 aufweist, und **dadurch gekennzeichnet, dass** man das Ammoniumformiat durch Hydrolyse von Methylformiat in Gegenwart des tertiären Amins A bereitstellt.

2. Verfahren nach Anspruch 1, **dadurch gekenn-**zeichnet, dass das tertiäre Amin A eine Pyridinverbindung ist.

3. Verfahren nach einem der vorhergehenden Ansprüche, **dadurch gekennzeichnet, dass** man die Zersetzung des Ammoniumformiats bei einer Reaktionstemperatur von nicht mehr als 130 °C durchführt.

4. Verfahren nach einem der vorhergehenden Ansprüche, **dadurch gekennzeichnet, dass** man die thermische Zersetzung als Destillation ausgestaltet, bei der Ameisensäure und gegebenenfalls Wasser aus dem Ammoniumformiat-haltigen Edukt abdestilliert.

5. Verfahren nach einem der vorhergehenden Ansprüche, **dadurch gekennzeichnet, dass** man die Hydrolyse des Methylformiats bis zu einem Umsatz im Bereich von 50 bis 95 % führt.

6. Verfahren nach Anspruch 5, **dadurch gekennzeichnet, dass** man das bei der Hydrolyse erhaltene Stoffgemisch einer ersten Destillation unterwirft, bei der man aus dem bei der Hydrolyse erhaltenen Stoffgemisch die Hauptmenge an Methanol und gegebenenfalls nicht umgesetztes Methylformiat abdestilliert, und den dabei erhaltenen Rückstand zur Zersetzung des Ammoniumformiats einer zweiten Destillation unterwirft, bei der man Ameisensäure und gegebenenfalls Wasser abdestilliert.

7. Verfahren nach einem der vorhergehenden Ansprüche, **dadurch gekennzeichnet, dass** man das tertiäre Amin A in die Reaktion zurückführt.

## Claims

1. A profess for preparing formic acid by thermally decomposing ammonium formates of tertiary amines A, wherein the tertiary amine A has a boiling point at atmospheric pressure in the range from 105 to 175°C and a $pK_a$ in water at 25°C in the range from 4 to 9, and wherein the ammonium formate is prepared by hydrolyzing methyl formate in the presence of the tertiary amine A.

2. The process according to claim 1, wherein the tertiary amine A is a pyridine compound.

3. The process according to either of the preceding claims, wherein the decomposition of the ammonium formate is carried out at a redaction temperature of not more than 130°C.

4. The profess according to any of the preceding claims, wherein the thermal decomposition is configured as a distillation in which formic acid and, where prevent, water are distilled out of the ammonium formate-containing reactant.

**5.** The process according to any of the preceding claims, wherein the hydrolysis of the methyl formate is carried out up to a conversion in the range from 50 to 95%.

**6.** The process according to claim 5, wherein the mixture obtained in the hydrolysis is subjected to a first distillation in which the majority of methanol and any unconverted methyl formate are distilled out of the mixture obtained in the hydrolysis, and the resulting residue is subjected to a second distillation to decompose the ammonium formate, in which formic acid and, where present, water are distilled off.

**7.** The process according to any of the preceding clams, wherein the tertiary amine A is recycled into the reaction.

**Revendications**

**1.** Procédé pour la préparation d'acide formique pair décomposition thermique de formiates d'ammonium d'amines tertiaires A, **caractérisé en ce que** l'amine tertiaire A présente un point d'ébullition à pression normale dans la plage de 105 à 175°C et une valeur $pK_a$ dans l'eau à 25°C dans la plage de 4 à 9, et **caractérisé en ce qu'**on prépare le formiate d'ammonium par hydrolyse de formiate de méthyle en présente de l'amine tertiaire A.

**2.** Procédé selon la revendication 1, **caractérisé en ce que** l'amine tertiaire A est un composé de pyridine.

**3.** Procédé selon l'une quelconque des revendication précédentes, **caractérisé en ce qu'**on réalise la décomposition du formiate d'ammonium à une température de réaction qui n'est pas supérieure à 130°C.

**4.** Procédé selon l'une quelconque des revendications précédentes, **caractérisé en ce qu'**on réaliste la décomposition thermique sous forme de distillation, dans laquelle on éliminé par distillation de l'acide formique et le cas échéant de l'eau à partir du produit de départ contenant du formiate d'ammonium.

**5.** Procédé selon l'une quelconque des revendications précédentes, **caractérisé en ce qu'**on conduit l'hydrolyse du formiate de méthyle jusqu'à une conversion dans la plage de 50 à 95%.

**6.** Procédé selon la revendication 5, **caractérisé en ce qu'**on soumet le mélange de substances obtenu lors de l'hydrolyse à une première distillation, dans laquelle on élimine du mélange de substances obtenu lors de l'hydrolyse la quantité principale de méthanol et le cas échéant du formiate de méthyle non transformé et on soumet le résidu ainsi obtenu, en vue de la décomposition du formiate d'ammonium, à une deuxième distillation, dans laquelle on élimine de l'acide formique et le cas échéant de l'eau.

**7.** Procédé selon l'une quelconque des revendications précédentes, **caractérisé en ce qu'**on recycle l'amine tertiaire A dans la réaction.

Figur 1:

**EP 1 784 375 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1432 A
- EP 563831 A
- GB 1028930 A
- US 3414610 A
- EP 95321 A
- EP 151510 A
- EP 357243 A
- DE 3428319 A